# EUROPEAN PATENT APPLICATION

(11) **EP 4 672 150 A1**
(43) Date of publication of application: **31.12.2025**
(21) Application number: 25183889.2
(22) Date of filing: 19.06.2025
(51) Int. Cl.: G06T 7/70, G06T 7/90, G06T 17/00

(54) **METHOD FOR DETERMINING OPTICAL PARAMETERS TO BE DISPLAYED ON A THREE-DIMENSIONAL MODEL**

(30) Priority: 24.06.2024 DK PA202470176
(71) Applicant: 3Shape A/S, 1060 Copenhagen K (DK)
(72) Inventor: JENSEN, Janus Nørtoft, 1060 Copenhagen K (DK); AANÆS, Henrik, 1060 Copenhagen K (DK); HOEDT, Asger Vejen, 1060 Copenhagen K (DK)
(74) Representative: Guardian IP Consulting I/S

(57) **Abstract**

The present disclosure relates to a method for determining a plurality of final optical parameters of a dental object in an intraoral cavity. The method may comprise receiving a plurality of two-dimensional images of the dental object, reconstructing a three-dimensional model of the dental object based on the plurality of two-dimensional images, determining camera positions of the plurality of two-dimensional images relative to the three-dimensional model, and receiving a plurality of optical parameters. The method may further include determining in an iterative manner: a plurality of simulated two-dimensional images of the dental object by inputting the plurality of optical parameters and the camera positions to a differentiable renderer, and multiple loss-values based on a loss function between the plurality of simulated two-dimensional images and the received plurality of two-dimensional images by adjusting the plurality of optical parameters. Additionally, the method may include determining a plurality of final optical parameters based on the adjusted plurality of optical parameters when a convergence criterion of the loss-values fulfils a convergence criterion.

## Description

### FIELD

The disclosure relates to an intraoral scanner system and a method for improving the visibility of a three-dimensional model of a dental object. More specifically, the disclosure relates to an improved method for determining a plurality of optical parameters based on a differentiable renderer.

### BACKGROUND

Intraoral scanners are electronic devices that may be used for, for example, capturing three-dimensional (3D) digital images of a dental object in an oral cavity. In an example, the intraoral scanners may include a light source that may project light rays onto a dental object to be scanned, such as teeth, gums, and other intraoral structures inside of patients' mouth. In an example, images captured by an intraoral scanner may be processed to generate digital impressions, such as a 3D surface model of oral cavity of a patient. The 3D surface model may be displayed on a screen for examination of the oral cavity or converted into a practical application by a three-dimensional printer system. For both purposes, there would be a need to further develop the surface representation of the 3D surface model even more trustworthy than today.

### SUMMARY

It is an aspect of the present disclosure to improve a surface representation of a three-dimensional surface model of a dental object f.x. by improving the representation of RGB-color, roughness, absorption, and scattering.

According to the aspect, a method for determining a plurality of final optical parameters of a dental object in an intraoral cavity is disclosed. The final optical parameters may include one or more of following parameters Red-Green-Blue (RGB) color, roughness scalar, absorption, and scattering. The method may comprise receiving a plurality of two-dimensional images of the dental object, and reconstructing a three-dimensional model of the dental object based on the plurality of two-dimensional images. The plurality of two-dimensional images may be captured by a handheld intraoral scanner, and the reconstructing of the 3D model of the dental object may be provided by one or more processors that may be arranged in one or more of following devices: a handheld intraoral scanner, an external computer relative to the handheld intraoral scanner, a cloud server or a regular sever that is arranged outside the handheld intraoral scanner. The method may further include determining camera positions of the plurality of two-dimensional images relative to the three-dimensional model and receiving a plurality of optical parameters. The camera positions of the plurality of two-dimensional images may be relative to the three-dimensional model of the dental object. The camera position may be determined by the reconstruction of the three-dimensional model of the dental object.

The plurality of optical parameters may include one or more of following includes one or more of following Red-Green-Blue (RGB) color parameter of a dental object, Roughness scalar parameter of a dental object, absorption parameter of a dental object, and scattering parameter of a dental object. The value of each of the plurality of optical parameters are to be adjusted by the method in an iterative manner based on a loss function between the plurality of simulated two-dimensional (2D) images and the received plurality of two-dimensional images. The camera positions of the plurality of two-dimensional images relative to the three-dimensional model are found and later used for determining the plurality of simulated 2D images.

The method may further include determining, in an iterative manner, a plurality of simulated two-dimensional images of the dental object by inputting the plurality of optical parameters and the camera positions to a differentiable renderer. The differentiable renderer is based on path tracing that traces propagation of light through a scene modelled by rays coming from the camera until reaching a light source.

The differential renderer may be used for determining optical parameters in an image or a volume. Differential rendering involves computing gradients of rendered images with respect to optical parameters, including color, roughness, absorption or scattering. This may be applied to adjust and optimize the optical parameters within an image or a volume based on specific objectives.

The differentiable renderer may be configured to perform path-tracing through pixels of a virtual camera to a light source via a dental object, and wherein the path-tracing is performed for each of the camera positions.

The differential renderer renders a scene to produce an image of that scene. This step involves simulating light transport and interaction with materials of that scene to determine the optical parameters of each pixel. An objective function may then be defined, which quantifies the difference between the rendered image and a target image. This function could measure pixel color, roughness absorption or scattering differences, structural similarities, or other image quality metrics. The differential renderer may then compute gradients of the objective function with respect to the optical parameters. These gradients indicate how changes in parameters, such as material colors or light intensities, affect the image. Using gradient-based optimization methods (like gradient descent), the scene parameters are adjusted iteratively to minimize the objective function. This process refines the optical parameters in the rendered image to better match the desired outcome.

The differential renderer may be based on inverse rendering which infers optical parameters, such as surface reflectance properties.

The method may further include determining, in the iterative manner, multiple loss-values based on a loss function between the plurality of simulated two-dimensional images and the received plurality of two-dimensional images by adjusting the plurality of optical parameters. After an iteration the plurality of optical parameters is adjusted for the purpose of minimizing the loss-value which would reflect a simulated two-dimensional image with optical parameters that are similar to or same as the plurality of received 2D images, i.e. the target image. The plurality of simulated two-dimensional images may correspond to the determined camera positions and includes the plurality of final optical parameters.

The method may include determining a plurality of final optical parameters based on the adjusted plurality of optical parameters when a convergence criterion of the loss-values fulfils a convergence criterion. The plurality of final optical parameters would include the adjusted plurality of optical parameters when the loss values are at a minimum or have fulfilled a quality criterion. The quality criterion may be determined by limited processing resources, the usage of the 3D model (diagnostic usages, designs of tooth wears, such as a crown, aligner etc.), or the quality criterion may be predetermined for obtaining the best quality irrespective of the usages of the 3D model.

Each of the multiple loss-values may include a pixel intensity difference between corresponding pixel of the plurality of simulated two-dimensional images and the received plurality of two-dimensional images.

A further aspect of the disclosure is to provide an intraoral scanning system that may be configured to determine a plurality of final optical parameters of a dental object. The intraoral scanning system may include a handheld intraoral scanner that may be configured to capture a plurality of two-dimensional images of a dental object. The system may further include one or more processors configured to reconstruct a three-dimensional model of the dental object based on the plurality of two-dimensional images. The one or more processors may be configured to determine camera positions of the plurality of two-dimensional images relative to the three-dimensional model. Furthermore, the one or more processors may be configured to determine plurality of simulated two-dimensional images of the dental object by inputting a plurality of optical parameters and the camera positions to a differentiable renderer. The plurality of optical parameters may be stored in a memory unit of the system. Additionally, the one or more processors may be configured to determine, in an iterative manner, multiple loss-values based on a loss function between the plurality of simulated two-dimensional images and the received plurality of two-dimensional images by adjusting the plurality of optical parameters. The one or more processors may be configured to determine a plurality of final optical parameters based on the adjusted plurality of optical parameters when a convergence criterion of the loss-values fulfils a convergence criterion.

The adjusting of the plurality of optical parameters may be based on a gradient of loss values that includes the multiple loss-values. The relation between the loss values in the gradient may be convex shaped with a single minimum. In this example the convergence criterion may be determined by the single minimum, which means the adjusted optical parameters that corresponds to a loss-value at the single minimum would be considered as the final optical parameters that is later applied to a three-dimensional model of the dental object.

The method may include determining, in the iterative manner, a gradient of loss-values based on the multiple loss-values, and when a convergence criterion of the gradient of loss-values fulfils a convergence criterion, the plurality of final optical parameters may be determined based on the adjusted plurality of optical parameters. **In** this example, the determining of the multiple loss-values stops when the convergence criterion is fulfilled.

The plurality of final optical parameters may be converted to optical representation parameters that are suitable for being displayed on a three-dimensional model. The method may include determining an optical representation parameter based on the plurality of final optical parameters, and displaying the three-dimensional model of the dental object including the optical representation parameter. The optical representation parameter may be one of the following:
- diffusely scattering parameter of the dental object,
- glossy reflection parameter of the dental object,
- translucent parameter of the dental object, and
- internal dental feature parameter of the dental object.

In another example, the method may include determining a plurality of optical representation parameters based on the plurality of final optical parameters, and displaying the three-dimensional model of the dental object including the plurality of optical representation parameters. The plurality of optical representation parameters may include one or more of the following:
- diffusely scattering parameter of the dental object,
- glossy reflection parameter of the dental object,
- translucent parameter of the dental object, and
- internal dental feature parameter of the dental object.

For example, the diffusely scattering parameter may be determined based on the color parameters. The glossy reflection parameter may be determined based on roughness scalar parameters. The translucent parameter may be determined based on the absorption and/or the scattering parameters. The internal dental feature parameters may be determined by absorption and scattering parameters when the received plurality of two-dimensional images includes infrared data. Infrared data may include acquired reflections from the dental object that include wavelengths between 800 nm to 1200 nm.

The method may further include displaying the three-dimensional model of the dental object including one or more of the multiple optical representation parameters. The surface of the displayed three-dimensional model may include the one or more optical representation parameters. In one example, the method may receive a user input via a user interface which determines which of the one or more optical representation parameters are to be displayed on the three-dimensional model.

The reconstructing of the three-dimensional model may be performed by the one or more processors in real time and during scanning of the dental object. The rendering of the three-dimensional model on a display unit may be performed in real time while scanning the dental object. The determining of the plurality of final optical parameters may be automatically performed while scanning the dental object, and the plurality of final optical parameters may be applied to the three-dimensional model during a post-processing of the three-dimensional model. The post-processing may be initiated automatically after completion of the scanning of the dental object, or, the post-processing may be initiated upon receiving a user input.

The plurality of two-dimensional images may include visible light information, infrared light information, ultraviolet light information or a combination of two or more of visible light information, infrared light information and ultraviolet light information. The intraoral scanning system may include multiple light sources that includes two or more of following light sources, a first light source configured to emit white light, a second light source configured to emit red light, a third light source configured to emit green light, a fourth light source configured emit blue light, a fifth light source configured to emit infrared light and a sixth light source configured to emit ultraviolet light. The one or more processors may be configured to switch between the different two or more light sources. The visible light information may include one or more wavelengths between 350 nm and 750 nm, the ultraviolet light information may include one or more wavelengths between 300 and 450 nm, and the infrared light information may include one or more wavelengths between 800 nm and 1200 nm.

The plurality of two-dimensional images may include infrared information which allows classification of internal dental features of the dental object. The internal dental features may be caries, interproximal caries, cracks, dentine, enamel and/or the dentine-enamel junction. **In** this example, the final optical parameters may correspond to scattering and absorption within the dental object, and by performing a differential rendering of the inside of the dental object would allow an improved visibility of the internal dental features, and thereby, an improved classification of the internal dental features. The method may comprise classifying parts of the dental object into an internal dental feature based on the plurality of final optical parameters that corresponds to infrared information in the plurality of two-dimensional images. The one or more processors may be configured to classify parts of the dental object into an internal dental feature based on the plurality of final optical parameters that corresponds to infrared information in the plurality of two-dimensional images.

The determined optical representation parameter may correspond to visible information in the plurality of two-dimensional images, and wherein the method further comprising optimizing the optical representation parameter of the dental object based on the classified parts of the dental object. For example, the appearance of the surface of a dental object may be affected by the internal dental features of the dental object, and therefore, it would be an advantage to utilize the knowledge of the internal dental features, via the classification, to further improve the coloring of the surface of the dental object. The further improvement of the coloring may be based on the classification of the internal dental features of the dental object.

Assuming the dental object is translucent then we need to estimate optical parameters that describes the behaviour of light inside the dental object. One way of describing this is through absorption and scattering coefficients. These parameters describe how likely light is to be absorbed (converted into heat) or scattered in a new direction. One approach to do this is by creating a volume around the dental object and estimate absorption and scattering coefficients for each voxel in the volume of the dental object. This may be accomplished by an iterative process where once the multiple loss-values has converged at a specific resolution of the volume, the volume is further subdivided into smaller voxel and new multiple loss-values are determined. The resolution of the volume in a first iteration may be 1x1x1 volume where we estimate one pair of final optical parameters, such as absorption and scattering parameters. Then, in another iteration the volume is further divided into 2x2x2 volume and estimate of eight pairs of final optical parameters are determined, in yet another iteration 64 pairs and henceforth. The method may include determining a volume around the dental object, wherein the volume includes multiple voxels, and then, determining, in an iterative manner the plurality of simulated two-dimensional images of the dental object by performing path-tracing via the differentiable renderer through the pixels of the virtual camera at each of the camera positions and through the multiple voxels of the volume, and the multiple loss-values of each of the volume between the plurality of simulated two-dimensional images and the received plurality of two-dimensional images by adjusting the plurality of optical parameters. The method may further include determining the plurality of final optical parameters based on the adjusted plurality of optical parameters when a convergence criterion of the loss-values fulfils a convergence criterion. Finally, the method may then subdivide the volume into more voxels and repeat the iterative determination of the plurality of simulated two-dimensional images and the multiple loss-values. The subdivision of the volume into more voxels ends when a wanted resolution of the volume is obtained. The wanted resolution may be predetermined.

To improve the visibility of an internal dental feature of a dental object it would be of an advantage to combine in a non-linear manner two-dimensional images that includes infrared wavelength(s), visible wavelengths and ultraviolet wavelengths. **In** some situations, the visibility of an internal dental feature of a dental object would be decent in a two-dimensional image which includes only infrared wavelength(s). Irrespective of the two-dimensional image includes a combination of infrared wavelengths with different wavelengths or only infrared wavelengths, it would be of an advantage to apply the method to optimize optical representation in a two-dimensional image only. In this example, the method includes receiving another plurality of two-dimensional images of the dental object, wherein the another plurality of two-dimensional images includes infrared wavelengths, and wherein the another plurality of two-dimensional images are captured at similar camera positions as the plurality of two-dimensional images. Furthermore, the method includes determining camera positions of the another plurality of two-dimensional images relative to the three-dimensional model, and the multiple loss-values is determined based on a loss function between the plurality of simulated two-dimensional images and the received another plurality of two-dimensional images by adjusting the plurality of optical parameters, and determining a plurality of final optical parameters based on the adjusted plurality of optical parameters when a convergence criterion of the loss-values fulfils a convergence criterion. Furthermore, the method may include rendering a two-dimensional image with the optical representation parameter and displaying the rendered two-dimensional image.

### BRIEF DESCRIPTION OF THE FIGURES

Aspects of the disclosure may be best understood from the following detailed description taken in conjunction with the accompanying figures. The figures are schematic and simplified for clarity, and they just show details to improve the understanding of the claims, while other details are left out. Throughout, the same reference numerals are used for identical or corresponding parts. The individual features of each aspect may each be combined with any or all features of the other aspects. These and other aspects, features and/or technical effect will be apparent from and elucidated with reference to the illustrations described hereinafter in which:
FIG. 1 illustrates a flow diagram of a method,
FIG. 2 illustrates another example of a flow diagram of a method,
FIGS. 3A and 3B illustrate examples of simulated two-dimensional images,
FIG. 4 illustrates an example of a method,
FIGS. 5A, 5B, and 5C illustrate an example of a method, and
FIG. 6 illustrates an example of an intraoral scanner system.

### DETAILED DESCRIPTION

The detailed description set forth below in connection with the appended drawings is intended as a description of various configurations. The detailed description includes specific details for the purpose of providing a thorough understanding of various concepts. However, it will be apparent to those skilled in the art that these concepts may be practiced without these specific details. Several aspects of the devices, systems, mediums, programs and methods are described by various blocks, functional units, modules, components, circuits, steps, processes, algorithms, etc. (collectively referred to as "elements"). Depending upon particular application, design constraints or other reasons, these elements may be implemented using electronic hardware, computer program, or any combination thereof.

The electronic hardware may include microprocessors, microcontrollers, digital signal processors (DSPs), field programmable gate arrays (FPGAs), programmable logic devices (PLDs), gated logic, discrete hardware circuits, and other suitable hardware configured to perform the various functionality described throughout this disclosure. Computer program shall be construed broadly to mean instructions, instruction sets, code, code segments, program code, programs, subprograms, software modules, applications, software applications, software packages, routines, subroutines, objects, executables, threads of execution, procedures, functions, etc., whether referred to as software, firmware, middleware, microcode, hardware description language, or otherwise.

A scanning for providing intra-oral scan data may be performed by a dental scanning system that may include an intraoral scanning device such as the TRIOS series scanners from 3 Shape A/S. The dental scanning system may include a wireless capability as provided by a wireless network unit. The scanning device may employ a scanning principle such as triangulation-based scanning, confocal scanning, focus scanning, ultrasound scanning, x-ray scanning, stereo vision, structure from motion, optical coherent tomography OCT, or any other scanning principle. In an embodiment, the scanning device is capable of obtaining surface information by operated by projecting a pattern and translating a focus plane along an optical axis of the scanning device and capturing a plurality of 2D images at different focus plane positions such that each series of captured 2D images corresponding to each focus plane forms a stack of 2D images. The acquired 2D images are also referred to herein as raw 2D images, wherein raw in this context means that the images have not been subject to image processing. The focus plane position is preferably shifted along the optical axis of the scanning system, such that 2D images captured at several focus plane positions along the optical axis form said stack of 2D images (also referred to herein as a sub-scan) for a given view of the object, i.e. for a given arrangement of the scanning system relative to the object. After moving the scanning device relative to the object or imaging the object at a different view, a new stack of 2D images for that view may be captured. The focus plane position may be varied by means of at least one focus element, e.g., a moving focus lens. The scanning device is generally moved and angled relative to the dentition during a scanning session, such that at least some sets of sub-scans overlap at least partially, to enable reconstruction of the digital dental 3D model by stitching overlapping 3D sub-scans together in real-time and display the progress of the virtual 3D model on a display as feedback to the user. The result of stitching is the digital 3D representation of a surface larger than that which can be captured by a single sub-scan, i.e. which is larger than the field of view of the 3D scanning device. Stitching, also known as registration and fusion, works by identifying overlapping regions of 3D surface in various sub-scans and transforming sub-scans to a common coordinate system such that the overlapping regions match, finally yielding the digital 3D model. An Iterative Closest Point (ICP) algorithm may be used for this purpose. Another example of a scanning device is a triangulation scanner, where a time varying pattern is projected onto the dental object and a sequence of images of the different pattern configurations are acquired by one or more cameras located at an angle relative to the projector unit.

Color texture of the dental object may be acquired by illuminating the object using different monochromatic colors such as individual red, green and blue colors or my illuminating the object using multichromatic light such as white light. A 2D image may be acquired during a flash of white light.

Generally, the process of obtaining surface information in real time of a dental object to be scanned requires the scanning device to illuminate the surface and acquire high number of 2D images. Typically, a high-speed camera is used with a framerate of 300-2000 2D frames pr second dependent on the technology and 2D image resolution. The high amount of image data needed to be handled by the scanning device to eighter directly forward the raw image data stream to an external processing device or performing some image processing before transmitting the data to an external device or display. This process requires that multiple electronic components inside the scanner is operating with a high workload thus requiring a high demand of current.

The scanning device comprises one or more light projectors configured to generate an illumination pattern to be projected on a three-dimensional dental object during a scanning session. The light projector(s) preferably comprises a light source, a mask signal having a spatial pattern, and one or more lenses such as collimation lenses or projection lenses. The light source may be configured to generate light of a single wavelength or a combination of wavelengths (mono- or polychromatic). The combination of wavelengths may be produced by using a light source configured to produce light (such as white light) comprising different wavelengths. Alternatively, the light projector(s) may comprise multiple light sources such as LEDs individually producing light of different wavelengths (such as red, green, and blue) that may be combined to form light comprising the different wavelengths. Thus, the light produced by the light source may be defined by a wavelength defining a specific color, or a range of different wavelengths defining a combination of colors such as white light. In an embodiment, the scanning device comprises a light source configured for exciting fluorescent material of the teeth to obtain fluorescence data from the dental object. Such a light source may be configured to produce a narrow range of wavelengths. In another embodiment, the light from the light source is infrared (IR) light, which is capable of penetrating dental tissue. The light projector(s) may be DLP projectors using a micro mirror array for generating a time varying pattern, or a diffractive optical element (DOF), or back-lit mask signal projectors, wherein the light source is placed behind a mask signal having a spatial pattern, whereby the light projected on the surface of the dental object is patterned. The back-lit mask signal projector may comprise a collimation lens for collimating the light from the light source, said collimation lens being placed between the light source and the mask signal. The mask signal may have a checkerboard pattern, such that the generated illumination pattern is a checkerboard pattern. Alternatively, the mask signal may feature other patterns such as lines or dots, etc.

The scanning device preferably further comprises optical components for directing the light from the light source to the surface of the dental object. The specific arrangement of the optical components depends on whether the scanning device is a focus scanning apparatus, a scanning device using triangulation, or any other type of scanning device. A focus scanning apparatus is further described in EP 2 442 720 B1 by the same applicant, which is incorporated herein in its entirety.

The light reflected from the dental object in response to the illumination of the dental object is directed, using optical components of the scanning device, towards the image sensor(s). The image sensor(s) are configured to generate a plurality of images based on the incoming light received from the illuminated dental object. The image sensor unit may be a high-speed image sensor such as an image sensor configured for acquiring images with exposures of less than 1/1000 second or frame rates in excess of 250 frames pr. second (fps). As an example, the image sensor may be a rolling shutter (CCD) or global shutter sensor (CMOS). The image sensor(s) may be a monochrome sensor including a color filter array such as a Bayer filter and/or additional filters that may be configured to substantially remove one or more color components from the reflected light and retain only the other non-removed components prior to conversion of the reflected light into an electrical signal. For example, such additional filters may be used to remove a certain part of a white light spectrum, such as a blue component, and retain only red and green components from a signal generated in response to exciting fluorescent material of the teeth.

The network unit may be configured to connect the dental scanning system to a network comprising a plurality of network elements including at least one network element configured to receive the processed data. The network unit may include a wireless network unit or a wired network unit. The wireless network unit is configured to wirelessly connect the dental scanning system to the network comprising the plurality of network elements including the at least one network element configured to receive the processed data. The wired network unit is configured to establish a wired connection between the dental scanning system and the network comprising the plurality of network elements including the at least one network element configured to receive the processed data.

The dental scanning system preferably further comprises a processor configured to generate scan data (such as extra-oral scan data and/or intra-oral scan data) by processing the two-dimensional (2D) images acquired by the scanning device. The processor may be part of the scanning device. As an example, the processor may comprise a Field-programmable gate array (FPGA) and/or an Advanced RISC Machines (ARM) processor located on the scanning device. The scan data comprises information relating to the three-dimensional dental object. The scan data may comprise any of: 2D images, 3D point clouds, depth data, texture data, intensity data, color data, and/or combinations thereof. As an example, the scan data may comprise one or more point clouds, wherein each point cloud comprises a set of 3D points describing the three-dimensional dental object. As another example, the scan data may comprise images, each image comprising image data e.g. described by image coordinates and a timestamp (x, y, t), wherein depth information can be inferred from the timestamp. The image sensor(s) of the scanning device may acquire a plurality of raw 2D images of the dental object in response to illuminating said object using the one or more light projectors. The plurality of raw 2D images may also be referred to herein as a stack of 2D images. The 2D images may subsequently be provided as input to the processor, which processes the 2D images to generate scan data. The processing of the 2D images may comprise the step of determining which part of each of the 2D images are in focus in order to deduce/generate depth information from the images. The internal depth information may be used to generate 3D point clouds comprising a set of 3D points in space, e.g., described by cartesian coordinates (x, y, z). The 3D point clouds may be generated by the processor or by another processing unit. Each 2D/3D point may furthermore comprise a timestamp that indicates when the 2D/3D point was recorded, i.e., from which image in the stack of 2D images the point originates. The timestamp is correlated with the z-coordinate of the 3D points, i.e., the z-coordinate may be inferred from the timestamp. Accordingly, the output of the processor is the scan data, and the scan data may comprise image data and/or depth data, e.g. described by image coordinates and a timestamp (x, y, t) or alternatively described as (x, y, z). The scanning device may be configured to transmit other types of data in addition to the scan data. Examples of data include 3D information, texture information such as infra-red (IR) images, fluorescence images, reflectance color images, x-ray images, and/or combinations thereof.

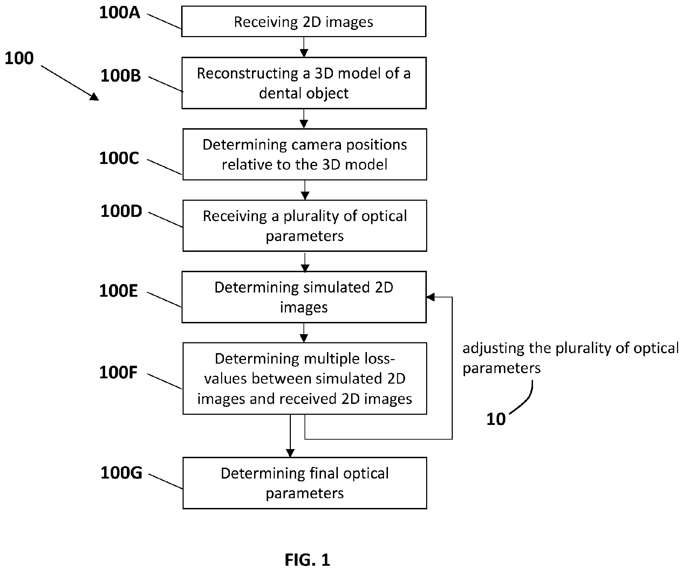

FIG. 1 illustrates a flow diagram of a method 100 for determining a plurality of final optical parameters of a dental object in an intraoral cavity. The method 100 includes receiving 100A a plurality of two-dimensional images of the dental object. In one example, the plurality of two-dimensional images may be captured by a handheld intraoral scanner and forwarded to one or more processors. The one or more processors may be arranged in the handheld intraoral scanner and/or an external computer/server. The method 100 including reconstructing 100B a three-dimensional model of the dental object based on the plurality of two-dimensional images. The plurality of two-dimensional images includes three-dimensional information, such as a pattern projected on the dental object by a light source of the handheld intraoral scanner. The pattern may be static or time-varying. The method 100 includes determining 100C camera positions of the plurality of two-dimensional images relative to the three-dimensional model. The plurality of two-dimensional images is captured by a camera of the handheld intraoral scanner at different camera positions, and these different camera positions are determined relative to the three-dimensional model by the one or more processors. In this specific example, the three-dimensional model of the dental object includes a plurality of optical parameters which improves the visibility of the three-dimensional model. The plurality of optical parameters may include red, green and blue color parameters, roughness scalar parameter, absorption parameter and/or scattering parameter. The method 100 is configured to determine a plurality of final optical parameters based on a differential renderer that is configured to receive the plurality of optical parameters 100D and the different camera positions. In an iterative manner 10 (100E,100F) a plurality of simulated two-dimensional images of the dental object is determined 100E by inputting the plurality of optical parameters and the camera positions to a differentiable renderer, and furthermore, multiple loss-values is determined 100F based on a loss function between the plurality of simulated two-dimensional images and the received plurality of two-dimensional images by adjusting the plurality of optical parameters. In each iteration 10, the plurality of optical parameters is adjusted for converging the multiple loss-values towards the convergence criterion. Furthermore, the method 100 includes determining 100G a plurality of final optical parameters based on the adjusted plurality of optical parameters when a convergence criterion of the loss-values fulfils a convergence criterion.

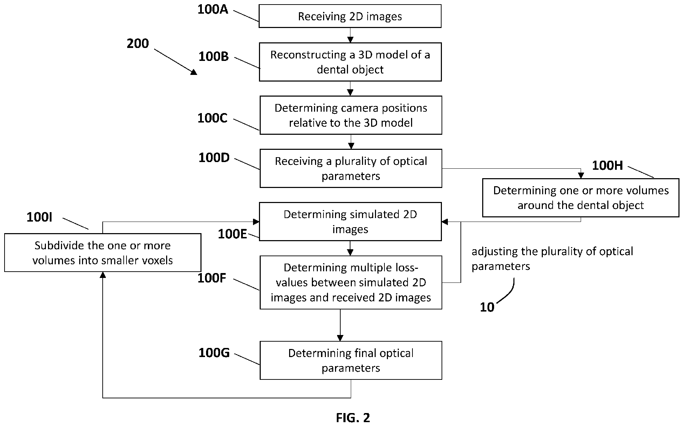

FIG.2 illustrates a flow diagram of a method 200 for determining a plurality of final optical parameters of a dental object in an intraoral cavity. The method 200 includes receiving 100A a plurality of two-dimensional images of the dental object. In one example, the plurality of two-dimensional images may be captured by a handheld intraoral scanner and forwarded to one or more processors. The one or more processors may be arranged in the handheld intraoral scanner and/or an external computer/server. The method 200 including reconstructing 100B a three-dimensional model of the dental object based on the plurality of two-dimensional images. The plurality of two-dimensional images includes three-dimensional information, such as a pattern projected on the dental object by a light source of the handheld intraoral scanner. The pattern may be static or time-varying. The method 200 includes determining 100C camera positions of the plurality of two-dimensional images relative to the three-dimensional model. The plurality of two-dimensional images is captured by a camera of the handheld intraoral scanner at different camera positions, and these different camera positions are determined relative to the three-dimensional model by the one or more processors. In this specific example, the three-dimensional model of the dental object includes a plurality of final optical parameters which improves the visibility of the three-dimensional model. The plurality of optical parameters may include red, green and blue color parameters, roughness scalar parameter, absorption parameter and/or scattering parameter. The method 200 includes determining a plurality of final optical parameters based on a differential renderer that is configured to receive the plurality of optical parameters 100D and the different camera positions. In this specific example, the method includes determining 100H a volume around the dental object, wherein the volume includes multiple voxels.

In an iterative manner 10 a plurality of simulated two-dimensional images of the dental object is determined 100E by inputting the plurality of optical parameters and the camera positions to a differentiable renderer, wherein a path-tracing is performed by the differentiable renderer through the pixels of a virtual camera at each of the camera positions and through the multiple voxels of the volume. Furthermore, multiple loss-values is determined 100F of each of the multiple voxels of the volume based on a loss function between the plurality of simulated two-dimensional images and the received plurality of two-dimensional images by adjusting the plurality of optical parameters,

In each iteration 10, the plurality of optical parameters is adjusted for converging the multiple loss-values towards the convergence criterion. Furthermore, the method 200 includes determining 100G a plurality of final optical parameters based on the adjusted plurality of optical parameters when a convergence criterion of the loss-values fulfils a convergence criterion. Furthermore, after the plurality of final optical parameters is determined 100G, the iteration continues by subdividing the volume into more voxels, and repeating the iterative determination 100E of the plurality of simulated two-dimensional images, the multiple loss-values 100F and new final optical parameters 100G.

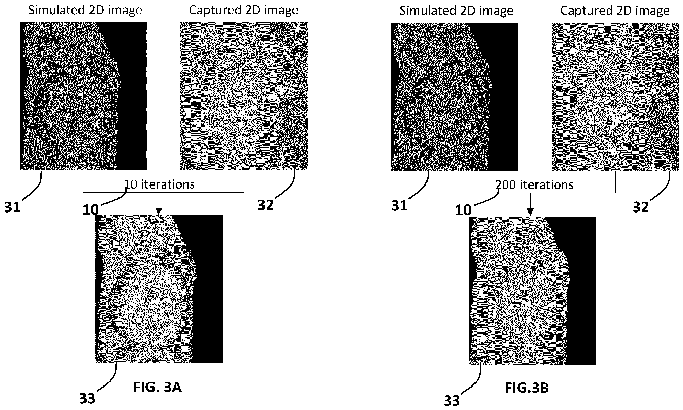

FIGS. 3A and 3B illustrate examples of simulated two-dimensional images before 31 and after 33 the iterations 10. In FIG. 3A, a simulated two-dimensional image 31 is seen with the initial optical parameters, i.e. before a first iteration. The target image in the differential renderer is the captured two-dimensional image 32 is seen. The final simulated two-dimensional image 33 includes the final optical parameter adjusted after 10 iterations. In FIG. 3B, the final simulated two-dimensional image 33 includes final optical parameters after 200 iterations. In both examples, the final optical parameters corresponds to one or more optical representation parameters, such as colors, diffuse, glossy, translucent or internal features. In this example, the final optical parameters corresponds to colors, diffuse and glossy.

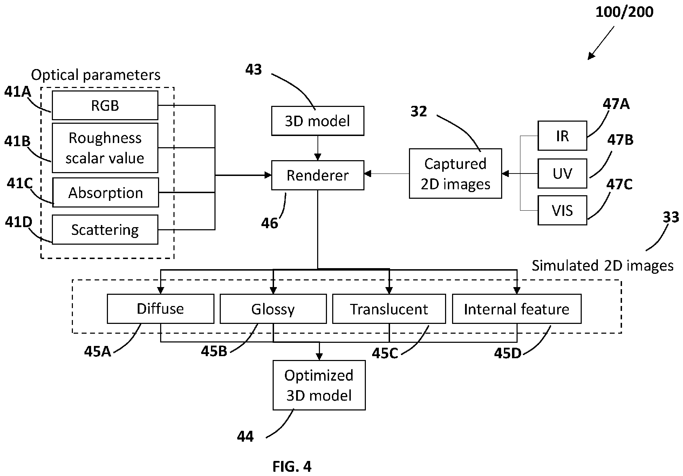

FIG. 4 illustrates an example of the method (100, 200). In this example, the differential renderer (100,200) receives one or more of the plurality of optical parameters (41A,41B,41C,41D), captured two-dimensional images 32 and a reconstructed 3D model 43 that is reconstructed based on the captured two-dimensional images 42. In this example, the two-dimensional images includes infrared wavelength(s) 47A, ultraviolet wavelength(s) 47B, visible wavelength(s) 47C or a combination of two or more of the wavelengths (47A, 47B, 47C). The differential renderer 46 outputs a plurality of simulated two dimensional images 33 after numerous of iterations 10 of the dental object, and wherein the dental object depicted in the plurality of simulated two-dimensional images includes improved diffuse scattering surfaces 45B which corresponds to red-green-blue optical parameters 41A. In another example, the differential renderer 46 outputs a plurality of simulated two dimensional images 33 after numerous of iterations 10 of the dental object, and wherein the dental object depicted in the plurality of simulated two-dimensional images 33 includes improved glossy effects 45B which corresponds to red-green-blue optical parameters 41A and the roughness scalar parameter 41B. In yet another example, the differential renderer 46 outputs a plurality of simulated two dimensional images 33 after numerous of iterations 10 of the dental object, and wherein the dental object depicted in the plurality of simulated two-dimensional images 33 includes improved translucent effects 45B which corresponds to absorption and scattering optical parameters (41C,41D). In a further example, the differential renderer 46 outputs a plurality of simulated two dimensional images 33 after numerous of iterations 10 of the dental object, and wherein the dental object depicted in the plurality of simulated two-dimensional images 33 includes improved visibility of internal dental features 45D which corresponds to absorption and scattering optical parameters (41C,41D) where the captured two-dimensional images 32 include infrared wavelength(s) 47A. In yet another example, the simulated two-dimensional images 33 includes a combination of the optical representation parameters (45A,45B,45C,45D).

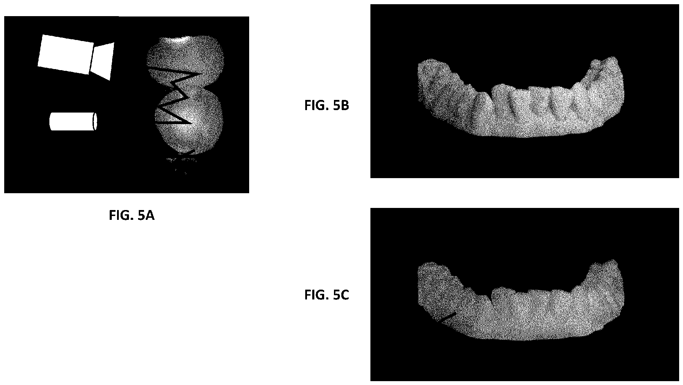

FIGS. 5A, 5B and 5C illustrate an example of the method (100, 200) where a volume 50 is applied around a dental object 53 for determining translucent and/or internal feature of the dental object 53. The volume is a three-dimensional square shaped box, but to the simplicity of FIG. 5A, the volume is seen as a two-dimensional square. In the example illustrated in FIG. 5A, the differential renderer performs path-tracing 54 within the volume of the dental object 53 starting from a virtual camera 51 having a camera position determined by a captured two-dimensional image 32 and ending at a virtual light source 52. Not seen in FIG. 5A, the volume 50 includes multiple voxels for which the rays in the path-tracing are intersecting for adjusting the optical parameters of the intersected multiple voxels. In an iterative manner the plurality of simulated two-dimensional images of the dental object is determined by performing path-tracing via the differentiable renderer through the pixels of the virtual camera at each of the camera positions and through the multiple voxels of the volume 50. Furthermore, in the iterative manner, the multiple loss-values of each of the multiple voxels of the volume 50 is determined between the plurality of simulated two-dimensional images and the received plurality of two-dimensional images by adjusting the plurality of optical parameters. Additionally, a plurality of final optical parameters is determined based on the adjusted plurality of optical parameters when a convergence criterion of the loss-values fulfils a convergence criterion, and to obtain a better resolution, the volume 50 is then further subdivided into more voxels, and the iteration is repeated for determining the plurality of simulated two-dimensional images and the multiple loss-values. FIG. 5B and 5C illustrate examples of an optimized three-dimensional model 55 where the volume 50 has been subdivided into a 2x2x2 volume 50, see FIG. 5B, and into 16x16x16, see FIG. 5C.

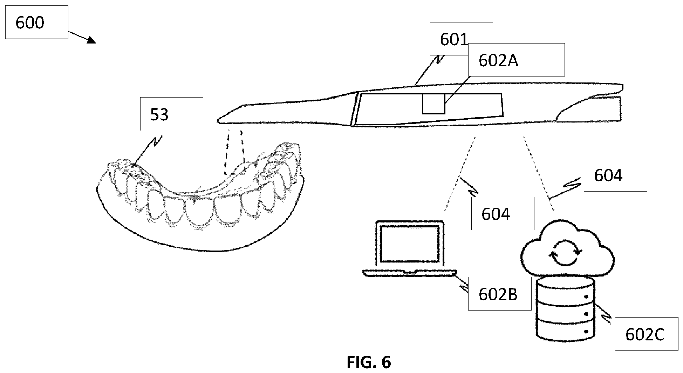

FIG. 6 illustrates an example of an intraoral scanner system 600. I this example, the system 600 includes a handheld intraoral scanner 601 scanning a dental object 53. The handheld intraoral scanner 601 is configured to capture a plurality of two-dimensional images of the dental object 53, The system 600 includes one or more processors (602A, 602B, 602C) configured to reconstruct a three-dimensional model of the dental object based on the plurality of two-dimensional images, and determine camera positions of the plurality of two-dimensional images relative to the three-dimensional model. In this example the scanner 601 is configured to communicated with external computers (602B,602C) via a wireless interface which is based on WIFI communication protocol and/or low energy Bluetooth communication protocol. In another example, the intraoral scanner 601 may be wired connected to external computers (602B, 602C). The one or more processors is further configured to determine, in an iterative manner a plurality of simulated two-dimensional images of the dental object by inputting the plurality of optical parameters and the camera positions to a differentiable renderer, multiple loss-values based on a loss function between the plurality of simulated two-dimensional images and the received plurality of two-dimensional images by adjusting the plurality of optical parameters, and determine a plurality of final optical parameters based on the adjusted plurality of optical parameters when a convergence criterion of the loss-values fulfils a convergence criterion.

### ITEM

1. A method for determining a plurality of final optical parameters of a dental object in an intraoral cavity, wherein the method comprising;
   - receiving a plurality of two-dimensional images of the dental object,
   - reconstructing a three-dimensional model of the dental object based on the plurality of two-dimensional images,
   - determining camera positions of the plurality of two-dimensional images relative to the three-dimensional model,
   - receiving a plurality of optical parameters,
   - determining in an iterative manner:
      o a plurality of simulated two-dimensional images of the dental object by inputting the plurality of optical parameters and the camera positions to a differentiable renderer,
      o multiple loss-values based on a loss function between the plurality of simulated two-dimensional images and the received plurality of two-dimensional images by adjusting the plurality of optical parameters, and
   - determining a plurality of final optical parameters based on the adjusted plurality of optical parameters when a convergence criterion of the loss-values fulfils a convergence criterion.
2. The method according to item 1, wherein the adjusting of the plurality of optical parameters is based on a gradient of loss values that includes the multiple loss-values.
3. The method according to any of the previous items, comprising determining, in the iterative manner, a gradient of loss-values based on the multiple loss-values, and when a convergence criterion of the gradient of loss-values fulfils a convergence criterion the plurality of final optical parameters is determined based on the adjusted plurality of optical parameters.
4. The method according to any of the previous items, wherein the plurality of simulated two-dimensional images corresponds to the determined camera positions and includes the plurality of final optical parameters.
5. The method according to any of the previous items, wherein the camera positions of the plurality of two-dimensional images are relative to the three-dimensional model of the dental object.
6. The method according to any of the previous items, comprising determining an optical representation parameter based on the plurality of final optical parameters, and displaying the three-dimensional model of the dental object including the optical representation parameter.
7. The method according to any of the previous items, comprising determining multiple optical representation parameters of a dental object based on different combinations of the plurality of final optical parameters.
8. The method according to item 7, comprising displaying the three-dimensional model of the dental object including one or more of the multiple optical representation parameters.
9. The method according to item 7, comprising selecting an optical representation parameter of the multiple optical representation parameters to be displayed on the three-dimensional model upon receiving a user input.
10. The method according to any of the previous items, wherein the plurality of optical parameters includes one or more of following:
   - Red-Green-Blue (RGB) color parameter of a dental object,
   - Roughness scalar parameter of a dental object,
   - Absorption parameter of a dental object, and
   - Scattering parameter of a dental object.
11. The method according to item 6, wherein the optical representation parameter includes:
   - diffusely scattering parameter of the dental object,
   - glossy reflection parameter of the dental object,
   - translucent parameter of the dental object, and
   - internal dental feature parameter of the dental object.
12. The method according to any of the previous items, wherein the plurality of two-dimensional images includes visible light information, ultraviolet information, infrared light information or a combination of the visible, ultraviolet and infrared light information.
13. The method according to item 12, wherein the visible light information includes one or more wavelengths between 350 nm and 750 nm, the infrared light information includes one or more wavelengths between 800 nm and 1100 nm, and the ultraviolet light information includes one or more wavelengths between 300 nm to 450 nm.
14. The method according to any of the previous items, wherein the plurality of final optical parameters is wavelength dependent.
15. The method according to any of the previous items, comprising classifying parts of the dental object into an internal dental feature based on the plurality of final optical parameters that corresponds to infrared information in the plurality of two-dimensional images.
16. The method according to item 6 and 15, comprising optimizing the optical representation parameter of the dental object based on the classified parts of the dental object, and wherein the determined optical representation parameter corresponds to visible information in the plurality of two-dimensional images.
17. The method according to items 15 or 16, wherein the internal dental feature includes one or more of following:
   - dentine,
   - enamel,
   - Dentine-Enamel-Junction (DEJ),
   - caries,
   - interproximal caries, and
   - cracks.
18. The method according to any of the items, wherein each of the multiple loss-values include a pixel intensity difference between corresponding pixel of the plurality of simulated two-dimensional images and the received plurality of two-dimensional images.
19. The method according to item 6, further comprises rendering the three-dimensional model with the optical representation parameter and displaying the three-dimensional model.
20. The method according to any of the previous items, wherein the differentiable renderer is configured to perform path-tracing through pixels of a virtual camera and to a light source via a dental object, and wherein the path-tracing is performed for each of the camera positions.
21. The method according to any of the previous claims, comprising:
   - determining a volume around the dental object, wherein the volume includes multiple voxels
   - determining, in an iterative manner:
      o the plurality of simulated two-dimensional images of the dental object by performing path-tracing via the differentiable renderer through the pixels of the virtual camera at each of the camera positions and through the multiple voxels of the volume, and
      o the multiple loss-values of the volume between the plurality of simulated two-dimensional images and the received plurality of two-dimensional images by adjusting the plurality of optical parameters,
   - determining the plurality of final optical parameters based on the adjusted plurality of optical parameters when a convergence criterion of the loss-values fulfils a convergence criterion, and
   - subdividing the volume into more voxels and repeat the iterative determination of the plurality of simulated two-dimensional images and the multiple loss-values.
22. A method for determining a plurality of final optical parameters of a dental object in an intraoral cavity, wherein the method comprising;
   - receiving a plurality of two-dimensional images of the dental object, wherein the plurality of two-dimensional images includes visible wavelengths,
   - reconstructing a three-dimensional model of the dental object based on the plurality of two-dimensional images,
   - receiving another plurality of two-dimensional images of the dental object, wherein the another plurality of two-dimensional images includes infrared wavelengths, and wherein the another plurality of two-dimensional images are captured at similar camera positions as the plurality of two-dimensional images
   - determining camera positions of the another plurality of two-dimensional images relative to the three-dimensional model,
   - receiving a plurality of optical parameters,
   - determining in an iterative manner:
      o a plurality of simulated two-dimensional images of the dental object by inputting the plurality of optical parameters and the camera positions to a differentiable renderer,
      o multiple loss-values based on a loss function between the plurality of simulated two-dimensional images and the received another plurality of two-dimensional images by adjusting the plurality of optical parameters, and
   - determining a plurality of final optical parameters based on the adjusted plurality of optical parameters when a convergence criterion of the loss-values fulfils a convergence criterion.
23. The method according to any of the previous items, further comprises rendering a two-dimensional image with the optical representation parameter and displaying the rendered two-dimensional image.
24. An intraoral scanner system that is configured for determining a plurality of final optical parameters of a dental object in an intraoral cavity, wherein the system comprises:
   - a handheld intraoral scanner configured to capture a plurality of two-dimensional images of the dental object,
   - one or more processors configure to:
      o reconstruct a three-dimensional model of the dental object based on the plurality of two-dimensional images,
      o determine camera positions of the plurality of two-dimensional images relative to the three-dimensional model,
      o determine, in an iterative manner:
         ▪ a plurality of simulated two-dimensional images of the dental object by inputting the plurality of optical parameters and the camera positions to a differentiable renderer,
         • multiple loss-values based on a loss function between the plurality of simulated two-dimensional images and the received plurality of two-dimensional images by adjusting the plurality of optical parameters, and
      o determine a plurality of final optical parameters based on the adjusted plurality of optical parameters when a convergence criterion of the loss-values fulfils a convergence criterion.

## Claims

1. A computer-implementable method for determining a plurality of final optical parameters of a dental object in an intraoral cavity, wherein the method comprising;
• receiving a plurality of two-dimensional images of the dental object,
• reconstructing a three-dimensional model of the dental object based on the plurality of two-dimensional images,
• determining camera positions of the plurality of two-dimensional images relative to the three-dimensional model,
• receiving a plurality of optical parameters,
• determining in an iterative manner:
o a plurality of simulated two-dimensional images of the dental object by inputting the plurality of optical parameters and the camera positions to a differentiable renderer,
o multiple loss-values based on a loss function between the plurality of simulated two-dimensional images and the received plurality of two-dimensional images by adjusting the plurality of optical parameters, and
• determining a plurality of final optical parameters based on the adjusted plurality of optical parameters when a convergence criterion of the loss-values fulfils a convergence criterion.

2. The method according to claim 1, wherein the adjusting of the plurality of optical parameters is based on a gradient of loss values that includes the multiple loss-values.

3. The method according to any of the previous claims, comprising determining, in the iterative manner, a gradient of loss-values based on the multiple loss-values, and when a convergence criterion of the gradient of loss-values fulfils a convergence criterion the plurality of final optical parameters is determined based on the adjusted plurality of optical parameters.

4. The method according to any of the previous claims, wherein the plurality of simulated two-dimensional images corresponds to the determined camera positions and includes the plurality of final optical parameters.

5. The method according to any of the previous claims, wherein the camera positions of the plurality of two-dimensional images are relative to the three-dimensional model of the dental object.

6. The method according to any of the previous claims, comprising determining an optical representation parameter based on the plurality of final optical parameters, and displaying the three-dimensional model of the dental object including the optical representation parameter.

7. The method according to any of the previous claims, comprising determining multiple optical representation parameters of a dental object based on different combinations of the plurality of final optical parameters.

8. The method according to claim 7, comprising displaying the three-dimensional model of the dental object including one or more of the multiple optical representation parameters.

9. The method according to any of the previous claims, wherein the differentiable renderer is configured to perform path-tracing through pixels of a virtual camera and to a light source via a dental object, and wherein the path-tracing is performed for each of the camera positions.

10. The method according to any of the previous claims, comprising:
• determining a volume around the dental object, wherein the volume includes multiple voxels
• determining, in an iterative manner:
o the plurality of simulated two-dimensional images of the dental object by performing path-tracing via the differentiable renderer through the pixels of the virtual camera at each of the camera positions and through the multiple voxels of the volume, and
o the multiple loss-values of the volume between the plurality of simulated two-dimensional images and the received plurality of two-dimensional images by adjusting the plurality of optical parameters,
• determining the plurality of final optical parameters based on the adjusted plurality of optical parameters when a convergence criterion of the loss-values fulfils a convergence criterion, and
• subdividing the volume into more voxels and repeat the iterative determination of the plurality of simulated two-dimensional images and the multiple loss-values.
